(19) Europäisches Patentamt
European Patent Office
Office européen des brevets

(11) **EP 4 279 025 A1**

(12) **EUROPEAN PATENT APPLICATION**
published in accordance with Art. 153(4) EPC

(43) Date of publication:
**22.11.2023 Bulletin 2023/47**

(51) International Patent Classification (IPC):
***A61F 2/01*** *(2006.01)*

(21) Application number: **22739137.2**

(86) International application number:
**PCT/CN2022/072103**

(22) Date of filing: **14.01.2022**

(87) International publication number:
**WO 2022/152262 (21.07.2022 Gazette 2022/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.01.2021 CN 202110053305**

(71) Applicant: **Suzhou Lavamed Co., Ltd.**
**Suzhou, Jiangsu 215123 (CN)**

(72) Inventors:
- **CUNNIFFE, Brendan**
  **Galway (IE)**
- **HUA, Xin**
  **Suzhou, Jiangsu 215123 (CN)**
- **SHEN, Quan**
  **Suzhou, Jiangsu 215123 (CN)**

(74) Representative: **Wenzel Nemetzade Warthmüller**
**Patentanwälte Part mbB**
**Maximilianstraße 2**
**80539 München (DE)**

(54) **THROMBUS REMOVAL APPARATUS**

(57)     A thrombus removal apparatus, comprising a body portion (1) and a distal portion (2). Both the body portion (1) and the distal portion (2) are self-expanding components, and the body portion (1) comprises a plurality of modules (13); each module (13) comprises a plurality of quadrilateral structures (14) formed by struts (16) in a circumferential direction, and two quadrilateral structures (14) adjacent to each other in the circumferential direction are connected at a quadrilateral structure connection node (15); proximal vertices (18) of each quadrilateral structure (14) of a proximal module (17) are connected to a proximal end (11) of the body portion (1) by means of respective connection struts (19), and proximal vertices (18) of each quadrilateral structure (14) of the remaining modules (13) are connected to, by means of the respective struts (16), one of distal vertices (20) of the adjacent modules (13) that are closer to the proximal end of the body portion (1), so that the adjacent modules (13) are connected by means of one or two connection nodes (3). By means of the connection mode of the thrombus removal apparatus, the apparatus can be delivered to a distal artery, and a large space is formed between the adjacent modules (13), thereby improving the thrombus capture and removal efficiency.

FIG. 3

EP 4 279 025 A1

**Description**

**Cross-Reference to Related Applications**

The present application claims the priority of Chinese Patent Application No. CN202110053305.9 filed on January 15, 2021, the disclosure of which is incorporated herein by reference in its entirety.

**Field of the Invention**

The present invention relates to medical device, and more particularly relates to a thrombus removal apparatus for capturing and removing, for example, thrombus or other obstruction formed or left in a patient's cranial artery.

**Background of the Invention**

Thrombus formed or accumulated in a patient's cranial artery may lead to acute ischemic stroke. Thrombus will obstruct blood flow in the affected arteries and cause irreparable damages to brain tissue, causing morbidity and even mortality of patients. At present, there are devices aimed at removing thrombus and restoring blood flow, and they have achieved varying degrees of success. Thrombus that has been formed is known to exist in various textures, which may be challenging for some devices. Usually, the thrombus removal apparatus cannot remove the whole thrombus at a time, which means that the blood perfusion in the affected artery cannot be restored. In order to remove enough thrombus and restore blood flow, the thrombus removal apparatus may need to pass for several times. Among them, the device needs to be pulled back towards the proximal side to the inlet of the aspiration catheter, re-sheathed in the microcatheter, and then pass through the thrombus again, redeployed and retracted. All this requires an operator to spend time and energy, and will prolong the time when a portion of the patient's brain is in a state of hypoxia.

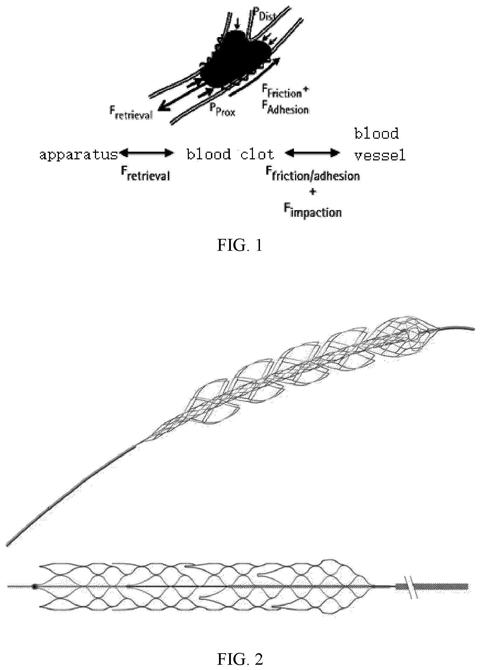

All mechanical thrombus removal devices must pass through the thrombus in a crimped manner through the microcatheter, and then they exit the microcatheter, radially expand to the deployed state and engage with the thrombus. Thrombus is infiltrated into the strut of the thrombus removal apparatus, so that the thrombus removal apparatus can grasp the thrombus; in this way, when the clinician pulls the thrombus removal apparatus, the thrombus is sufficiently grasped to be removed. The vast majority of commercial thrombus removal apparatus are engaged with thrombus by radial expansion when exiting the microcatheter. Their effectiveness in infiltrating and grasping thrombus mainly depends on each cell area size of the stent structure (i.e., the size of the opening space between adjacent struts) and the radially outward force generated by the structure. As long as the force exerted on the thrombus by the thrombus removal apparatus is greater than the force exerted on the thrombus by the blood vessel, the thrombus will be removed, as shown in Figure 1:

$$\mathrm{F_{retrieval}} > \mathrm{F_{resistant}}$$

As can be seen from the figure, the force exerted by blood vessels on thrombus or blood clot has two elements, namely, $\mathrm{F_{resistant}} = \mathrm{F_{friction}} + \mathrm{F_{impaction}}$, wherein $\mathrm{F_{friction}}$ is the force caused by the "stickiness" of the thrombus to the blood vessel wall, and $\mathrm{F_{impaction}}$ is the force caused by the blood pressure difference (from proximal end to distal end) across the thrombus. For the current commercial instruments, there are two completely different methods to integrate and remove thrombus. Second-generation instruments (such as Medtronic Solitaire, Stryker Trevo), which are now regarded as "original" instruments, generate radial force to fully penetrate the thrombus, and obtain a firm clamping force to grasp the thrombus when it is removed by rolling or dragging. This effectively captures the thrombus between the blood vessel wall and the stent, and the thrombus can effectively "roll" towards the proximal side during the retraction of the stent. The greater the radial force, the greater the clamping force on the thrombusis, but the greater the friction between the thrombus and the blood vessel wall is, because the thrombus pushes against the blood vessel wall with greater force. If the radial force of the structure is inherently too high, this mechanism for thrombectomy may be counterproductive. Third-generation instruments (such as Cerenovous Embotrap II, MicroVention ERIC) remove blood clots through more pushing actions. They are designed to have a large space between the modules of the stent, into which the thrombus will penetrate, as shown in Figure 2, so that the force exerted on the thrombus is mainly tangential to the blood vessel wall (in the proximal axial direction), without the same component of radially outward force acting on the thrombus. This serves to limit the wall friction force experienced by the thrombus due to stent deployment, and aims to make the thrombus easier to be removed.

Generally, these third-generation instruments are formed around a central spine wire, which limits the crimped contour of the structure; or a large number of connections are used in the whole instrument, which will adversely affect the bending stiffness of the structure, as shown in Figure 2.

In addition, in prior art, for the structure with capture net at the distal end, the thrombus will be contained inside the distal capture net from the inside, because under this structure, the thrombus will move towards the distal end from the space between the inner body and the outer body.

## Summary of the Invention

An objective of the invention is to provide a thrombus removal apparatus for removing thrombus formed or left in a patient's cranial artery, which intends to optimize structural competence so as to penetrate into as more thrombus as possible.

To achieve the objective, the present invention provides such a solution: a thrombus removal apparatus, comprising a main body part and a distal part, the main body part and the distal part being both self-expanding members, the utmost distal end of the main body part being connected with the utmost proximal end of the distal part, wherein the main body part comprises a plurality of modules; each module comprises a number of quadrilateral structures formed by struts along a circumferential direction, and any two adjacent quadrilateral structures along the circumferential direction are connected at a quadrilateral structure connecting node; an utmost proximal end vertex of each quadrilateral structure of the utmost proximal end module is connected to an utmost proximal end of the main body part via respective connecting struts, and an utmost proximal end vertex of each quadrilateral structure of the other modules is connected to one of the distal end vertices of the adjacent modules closer to the utmost proximal end of the main body part via respective connecting struts, so that any two adjacent modules are connected via one or two connecting nodes.

Preferably, the quadrangular structure is a rhombic structure.

Preferably, the module comprises four quadrilateral structures along the circumferential direction.

Preferably, when there is a connecting node between two adjacent modules, except the utmost proximal end module, the utmost proximal end vertex of each quadrilateral structure of the other modules is connected to the same distal end vertex of the adjacent modules closer to the utmost proximal end of the main body part via the respective connecting struts.

Preferably, when there are two connecting nodes between two adjacent modules, except the utmost proximal end module, the utmost proximal end vertices of two adjacent quadrangular structures of the other modules are connected to one of the distal end vertices of the adjacent modules closer to the utmost proximal end of the main body part via their respective connecting struts, and the utmost proximal end vertices of other adjacent quadrilateral structures are connected to another one distal vertex of the adjacent module closer to the utmost proximal end of the main body part via the respective connecting struts.

Preferably, three or four modules are provided.

Preferably, the main body part and the distal part are made of shape memory materials.

Preferably, the main body part is made by laser cutting a nickel-titanium alloy tube, expanding it to a required diameter on a forming mandrel, and then shaping it at the diameter.

Preferably, the distal part has a spherical structure.

Preferably, the distal part has a rugby-shaped structure.

Preferably, the distal part comprises a plurality of distal end longitudinal struts with the same geometry, one ends of all the distal longitudinal struts are connected together at the utmost proximal end of the distal part, and the other ends of all the distal end longitudinal struts are connected together at the utmost distal end of the distal part; the distal end longitudinal struts are distributed along the circumferential direction.

Preferably, the distal end longitudinal struts are evenly distributed along the circumferential direction.

Preferably, the projection of any distal end longitudinal strut on a plane passing through the axis of the distal part has a sinusoidal shape, so that the radial expansion of the distal part is accompanied by the rotational movement of the distal end longitudinal strut.

Preferably, the grid density of the distal part is greater than that of the main body part.

Preferably, the distal part (2) comprises six distal end longitudinal struts (23) with the same geometry.

The main body part of the thrombus removal apparatus provided by the invention basically has a modular design, and a plurality of duplicate modules are used to construct the whole stent structure. The space between adjacent modules of the main body part is maximized as much as possible, so that thrombus can easily infiltrate into the structure. Different from other modular designs formed around the central spine or connected with a large number of connection nodes, the invention has the minimal connection nodes between adjacent modules, and the number of the connecting nodes is one or two, thus providing a design with excellent bending stiffness characteristics compared with other mechanical thrombectomy instruments. This connecting mode provides excellent flexibility for the whole structure, enhances the ability of each individual module to act independently in the bending process, and at the same time improves the ability to be delivered into the more distal artery and the ability to be retrieved by pulling it back towards the proximal end into the aspiration catheter.

**Brief Description of the Drawings**

To illustrate the embodiments of the present invention or the technical solutions in prior art more clearly, the drawings used in the description of the embodiments or the prior art will be briefly described below. Apparently, the drawings in the following description are only some embodiments of the present invention.

Fig. 1 shows the forces acting on a thrombus in an artery;

Fig. 2 shows a schematic structural diagram of a thrombus removal apparatus in prior art;

Fig. 3 is a schematic structural view of a thrombus removal apparatus according to an embodiment of the present invention;

Fig. 4 is a structural development view of the main body part in fig. 3;

Fig. 5 is a schematic structural view of the distal part in fig. 3;

Fig. 6 is a schematic structural view of a main body part according to another embodiment of the present invention;

Fig. 7 is an expanded view of the main body part according to fig. 6.

Notes of Reference Numerals

| Reference numerals | Name | Reference Numerals | Name |
|---|---|---|---|
| 1 | main body part | 2 | distal part |
| 11 | utmost proximal end of the main body part | 12 | utmost distal end of the main body part |
| 21 | utmost proximal end of the distal part | 22 | utmost distal end of the distal part |
| 13 | module | 14 | quadrilateral structure |
| 15 | connecting nodes of the quadrilateral structure | 16 | strut |
| 17 | utmost proximal end module | 18 | utmost proximal end vertex of quadrilateral structure |
| 19 | connecting strut | 20 | distal end vertex of module |
| 3 | connecting node | 23 | longitudinal strut at distal end |

**Detailed Description of the Invention**

Hereinafter, the technical solutions in the embodiments of the invention will be described with reference to the accompanying drawings. Apparently, the described embodiments are only part of the embodiments of the invention, but not all embodiments. Based on the embodiments of the present invention, all other embodiments that can be obtained by those skilled in the art without inventive efforts fall within the scope of protection of the present invention.
It is noted that when an element is described to be "connected" to another element, it can be directly connected to said another element or there may be an intermediate element at the same time. In the field of interventional medical devices, the end of a medical device implanted in a human body or an animal body that is proximal to the operator is generally referred to as "proximal end" and the end that is distant from the operator is referred to as "distal end", whereby the "proximal end" and "distal end" of any part of the medical device are defined. In addition, the drawings are simplified and use non-exact scales, which are only used to facilitate and clearly illustrate the embodiments of the present invention. In addition, the technical solutions or technical features of various embodiments can be combined with each other within a reasonable extent.

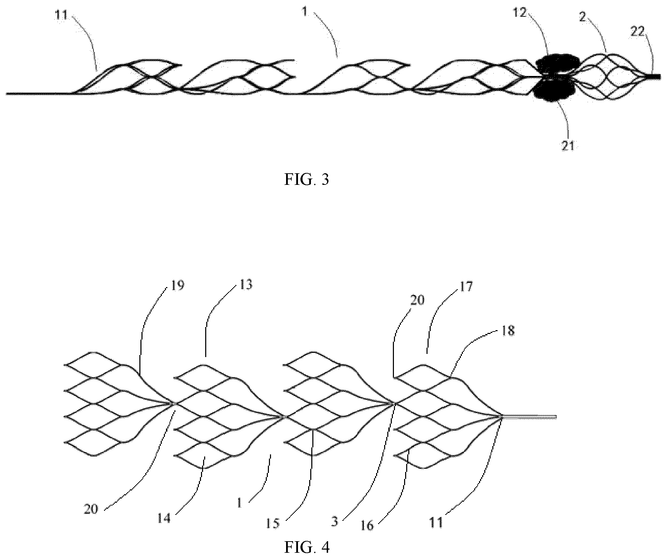

As shown in Fig. 3, Fig. 4 and Fig. 6-7, the invention discloses a thrombus removal apparatus, which comprises a main body part 1 and a distal part 2, wherein the main body part 1 and the distal part 2 are both self-expanding members, the utmost distal end 12 of the main body part is connected to the utmost proximal end 21 of the distal part. The main body part 1 has a stent structure, and the stent structure comprises a plurality of duplicate modules 13. The module 13 comprises a plurality of quadrilateral structures 14 formed by struts 16 along the circumferential direction, two adjacent quadrilateral structures 14 along the circumferential direction have a quadrilateral structure connecting node 15, and the utmost proximal end vertex 18 of each quadrilateral structure of the utmost proximal end module 17 is connected to the utmost proximal end 11 of the main body part through respective connecting struts 19. The proximal end vertex 18 of each quadrilateral structure of the remaining modules is connected to one of the distal end vertices 20 of the adjacent modules closer to the utmost proximal end of the main body part through respective connecting struts 19, and the adjacent modules are connected via one or two connecting nodes 3. The utmost proximal end module 17 is the module closest to the operator. A connecting node 3 coincides with a distal end vertex 20.

In a preferred embodiment, the quadrilateral structure is a rhombic structure.

Like all mechanical thrombus removal instruments, the device of the present invention is delivered to the required position for the thrombus in the cranial artery via a microcatheter, and the main body part 1 and the distal part 2 are self-expanding members, which are in a compressed and crimped state when the device is pulled into the microcatheter. The main body part 1 and the distal part 2 of the structure are pushed out of the microcatheter at the required position of the cranial artery, and both of them try to restore from the deformation experienced during the crimping due to the superelastic properties of shape memory materials such as nitinol. In other words, it tries to restore its expanded unstressed shape. The connecting mode of the device of the invention can ensure that the structure can be completely pulled into the microcatheter. Wherein the utmost proximal end vertices 18 of all quadrilateral structures on the module 13 are connected in some way to one of the distal end vertices 20 of the adjacent module closer to the utmost proximal end of the main body part.

As can be seen from the above, the main body part 1 is basically modular in design, and a plurality of duplicate modules 13 are used to construct the whole stent structure. The significance of the geometric structure of the main body part 1 is that it aims to optimize the thrombus pushing ability of the structure by maximizing the space between adjacent struts, and at the same time provide a design with excellent bending stiffness characteristics compared with other mechanical thrombus removal instruments. The minimal metal-to-artery nature of the structure allows a low (or small) crimped contour and enables the structure to be delivered to utmost distant cranial arteries, such as M3 and M4 branches. In addition, some published literature shows that this minimal metal-to-artery ratio improves the clinical performance because it reduces the vascular trauma during retrieval. The structure of the present invention maximizes the space between adjacent modules 13 as much as possible, so that thrombus can easily infiltrate into the stent structure. These modules can be connected together via a minimum of connecting nodes, such as one or two connecting nodes 3, depending on the specific stent configuration, as shown in Figures 4, 6 and 7.

As shown in Fig. 4, in one embodiment of the present invention, the module 13 includes four quadrilateral structures 14 along the circumference. When there is a connecting node 3 between two adjacent modules 13, except the utmost proximal end module 17, the utmost proximal end vertex 18 of each quadrilateral structure of the remaining modules 13 is connected to the same distal end vertex 20 of the adjacent module closer to the utmost proximal end of the main body part via respective connecting struts 19. The radial force of each module 13 (and the whole structure) depends on the geometric shape and material characteristics of these quadrilateral structures 14. This connecting mode provides excellent flexibility for the whole structure, because it enhances the ability of each individual module 13 to function independently in the bending process. Moreover, since the structure is not shaped along the central spine, unlike traditional thrombus removal apparatuses, it minimizes the amount of metal in the structure, thus leading to a very low-contour of crimped structure, which means that the structure can be compatible with a microcatheter with a 0.020- inch ID at most.

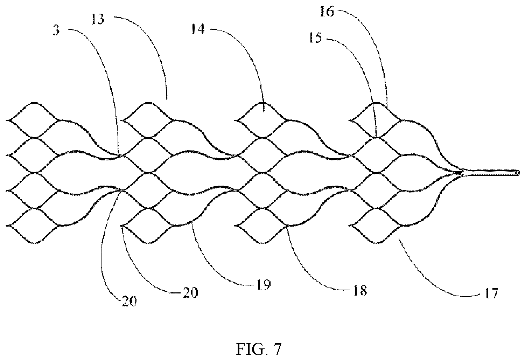

As shown in Fig. 7, in another embodiment, the module 13 includes four quadrilateral structures 14 along the circumference. When there are two connecting nodes 3 between two adjacent modules 13, except the utmost proximal end module 17, the utmost proximal end vertices 18 of two adjacent quadrilateral structures of the remaining modules are connected to one of the distal end vertices 20 of the adjacent modules closer to the utmost proximal end of the main body part via respective connecting struts 19, and the utmost proximal end vertices 18 of the other two adjacent quadrilateral structures are connected to another distal end vertex 20 of the adjacent module closer to the utmost proximal end of the main body part through respective connecting struts 19. In this embodiment, there are two connecting nodes 3 between adjacent modules 13. Other connection modes between two adjacent modules are also feasible.

The thrombus removal apparatus according to the present invention can be delivered to a farther distant distal artery, and is also convenient to be withdrawn by pulling back towards the proximal end into the aspiration catheter. The invention has the minimal connecting nodes, such as one or two, between adjacent modules 13, and this modular structure can realize the following functions:

1) Infiltrate thrombus, engaging with thrombus and retaining thrombus during retrieval.

2) There is a large space between adjacent modules, and in the process of removing thrombus, thrombus can infiltrate into the space and be contained in it.

3) Minimize the contact area between the stent structure and the blood vessel wall to minimize the possibility of blood vessel trauma.

4) Its connecting mode (i.e., one or two connecting nodes) provides enhanced flexibility and allows better traceability (during the movement to the thrombus position) and better retrievability (during the movement of the deployed structure to the proximal end).

The number of the modules 13 can be varied to form devices with different lengths. For example, when the length of the main body part is 40mm, it can include 4 modules, and when the length of the main body part is 30mm, it can include 3 modules, or alternatively it can be of other suitable lengths and has other suitable number of modules.

The main body part 1 and the distal part 2 are made of a shape memory material, such as nitinol. When preparing the main body part 1, the nickel-titanium alloy tube can be laser-cut, expanded to an appropriate "free diameter" on the forming mandrel, that is, a required diameter, and then shaped at that diameter.

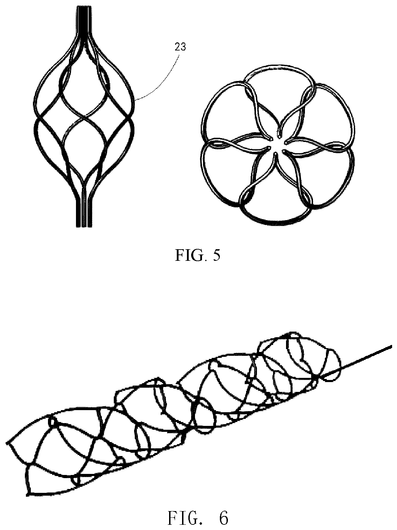

As shown in Figs. 3 and 5, the distal part 2 has a spherical structure, preferably a rugby-shaped structure, and may be an additional discrete component as an "all-round capture structure" of thrombus material not properly engaged with the strut 16 constituting the main body part 1 of the thrombus removal apparatus. That is, the distal part 2 serves to collect any thrombus material that is not engaged with the strut 16 of the main body part 1. In actuality, it is performed in a manner similar to an embolic protection structure by collecting any loose thrombus that has not been captured by the main body part of the thrombus removal apparatus.

The spherical structure of the distal part 2 comprises a plurality of distal end longitudinal struts 23 with the same geometry, one ends of all distal end longitudinal struts 23 are connected together at the utmost proximal end 21 of the distal part, and the other ends of all distal end longitudinal struts 23 are connected together at the utmost distal end 22 of the distal part, and the distal end longitudinal struts 23 are fixedly connected together only at the utmost proximal end 21 and the utmost distal end 22 of the distal part. The distal end longitudinal struts 23 are arranged along the circumference with intervals, preferably at uniform intervals. The shape of the projection of any distal end longitudinal strut 23 on a plane can be sinusoidal shape, which makes the distal part 2 expand radially with the rotational movement of the distal end longitudinal strut 23, for example, as illustrated in Figure 5. The radial expansion accompanied by rotational movement makes the thrombus easier to be captured and does not cause great pressure on the blood vessel wall. The distal end longitudinal strut 23 has no other connecting nodes except the two nodes at the utmost proximal end 21 and the utmost distal end 22 of the distal part.

In one embodiment of the present invention, the distal part 2 comprises six distal end longitudinal struts 23 having the same geometry. The grid density of the distal part 2 is greater than that of the main body part 1, so that the captured thrombus is always contained outside the distal part, that is, in the space formed between the main body part 1 and the proximal end of the rugby structure (see Fig. 3). Due to the high density of struts forming the rugby structure, it will be difficult for thrombus material to pass through the "net" formed by struts (see "axial view" in Fig. 5). This means that most of the thrombus material is likely to be captured at the proximal end of the rugby structure, so it will be pushed in front of the rugby structure when it is taken out.

As an embolic protection structure, the distal part 2 of the present invention is different from the traditional embolic protection structure in which thrombus material is captured inside a basket structure. In the present invention, thrombus is always contained outside the spherical structure itself for capturing any clot that has fallen off from the initial thrombus structure and may attempt to move towards the distal end to other blood vessels. If the thrombus passes towards the distal end, it will be captured by the distal end longitudinal strut 23 at the utmost proximal end of the spherical structure, contained outside the spherical structure itself, and pushed in front of the spherical structure when it is withdrawn towards the proximal end into the suction catheter. The distal part 2 serves as an "all-round capture structure" for the thrombus removal process, ensuring that all thrombus materials at the proximal end of the distal part 2 are pushed towards the proximal end when the structure is pulled back toward the aspiration catheter.

The spherical structure of the distal part 2 can perform its function as an independent component, but when the main body part 1 works together with the spherical structure, the overall efficiency of the combined main body-spherical structure as a thrombus removal apparatus will be greatly improved.

What have been described above are only the preferred embodiment of the present invention, which do not limit the scope of the present invention. Any equivalent structural modifications made by using the contents of the specification and drawings of the present invention or direct/indirect application in other related technical fields under the inventive concept of the present invention are included in the protection scope of the present invention.

**Claims**

1. A thrombus removal apparatus, comprising a main body part (1) and a distal part (2), the main body part (1) and the distal part (2) being both self-expanding members, an utmost distal end (12) of the main body part (1) being connected with an utmost proximal end (21) of the distal part (2), wherein the main body part (1) comprises a plurality of modules (13); each module (13) comprises a number of quadrilateral structures (14) formed by struts (16) along a circumferential direction, and any two adjacent quadrilateral structures (14) along the circumferential direction are connected at a quadrilateral structure connecting node (15); an utmost proximal end vertex (18) of each quadrilateral structure of the utmost proximal end module (17) is connected to the utmost proximal end (11) of the main body part via respective connecting struts (19), and an utmost proximal end vertex (18) of each quadrilateral structure of the other modules is connected to one of the distal end vertices (20) of the adjacent modules closer to the utmost proximal end of the main body part via respective connecting struts (19), so that adjacent modules are connected via one or two connecting nodes (3).

2. The thrombus removal apparatus of claim 1, wherein the quadrangular structure is a rhombic structure.

3. The thrombus removal apparatus of claim 1 or 2, wherein the module (13) comprises four quadrilateral structures (14) along the circumferential direction.

4. The thrombus removal apparatus of any of claims 1-3, wherein when there is one connecting node (3) between two adjacent modules (13), except the utmost proximal end module (17), the utmost proximal end vertex (18) of each quadrilateral structure of the other modules (13) is connected to the same distal end vertex (20) of the adjacent modules closer to the utmost proximal end of the main body part via the respective connecting struts (19).

5. The thrombus removal apparatus of any of claims 1-3, wherein when there are two connecting nodes (3) between two adjacent modules (13), except the utmost proximal end module (17), the utmost proximal end vertices (18) of two adjacent quadrangular structures of the other modules are connected to one of the distal end vertices (20) of the adjacent modules closer to the utmost proximal end of the main body part via their respective connecting struts (19), and the utmost proximal end vertices (18) of other adjacent quadrilateral structures are connected to another one distal vertex (20) of the adjacent module closer to the utmost proximal end of the main body part via the respective connecting struts (19).

6. The thrombus removal apparatus of any of claims 1-5, wherein three or four modules (13) are provided.

7. The thrombus removal apparatus of claim 1, wherein the main body part (1) and the distal part (2) are made of shape memory materials.

8. The thrombus removal apparatus of claim 7, wherein the main body part (1) is made by laser cutting a nickel-titanium alloy tube, expanding it to a required diameter on a forming mandrel, and then shaping it at the diameter.

9. The thrombus removal apparatus of claim 1, wherein the distal part (2) has a spherical structure.

10. The thrombus removal apparatus of claim 9, wherein the distal part (2) has a rugby-shaped structure.

11. The thrombus removal apparatus of claim 9 or 10, wherein the distal part (2) comprises a number of distal end longitudinal struts (23) with the same geometry, one ends of all the distal longitudinal struts (23) are connected together at the utmost proximal end (21) of the distal part, and the other ends of all the distal end longitudinal struts (23) are connected together at the utmost distal end (22) of the distal part; the distal end longitudinal struts (23) are distributed along the circumferential direction.

12. The thrombus removal apparatus of claim 11, wherein the distal end longitudinal struts (23) are evenly distributed along the circumferential direction.

13. The thrombus removal apparatus of claim 12, wherein the projection of any distal end longitudinal strut (23) on a plane passing through the axis of the distal part (2) has a sinusoidal shape, so that the radial expansion of the distal part is accompanied by the rotational movement of the distal end longitudinal strut (23).

14. The thrombus removal apparatus of any one of claims 9-12, wherein the density of grids of the distal part (2) is greater than that of the main body part (1).

15. The thrombus removal apparatus of any one of claims 9-12, wherein the distal part (2) comprises six distal end longitudinal struts (23) with the same geometry.

FIG. 1

FIG. 2

FIG. 3

FIG. 4

FIG. 5

FIG. 6

FIG. 7

## INTERNATIONAL SEARCH REPORT

| | |
|---|---|
| | International application No. |
| | **PCT/CN2022/072103** |

| A. | CLASSIFICATION OF SUBJECT MATTER |
|---|---|

A61F 2/01(2006.01)i

According to International Patent Classification (IPC) or to both national classification and IPC

| B. | FIELDS SEARCHED |
|---|---|

Minimum documentation searched (classification system followed by classification symbols)

A61B17; A61F2

Documentation searched other than minimum documentation to the extent that such documents are included in the fields searched

Electronic data base consulted during the international search (name of data base and, where practicable, search terms used)

CNTXT; WPABSC; DWPI; ENTXTC; CNKI: 血栓, 颅动脉, 阻塞, 中风, 捕获, 捕捉, 取出, 过滤, 多, 重复, 单元, 模块, 结构, 支架, 网格, 网, 杆, 丝, occlu+, embolic, artery, vessel, thrombus, thrombectomy, 徕瑞

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 112842467 A (SUZHOU LAVA MEDICAL TECHNOLOGY CO., LTD.) 28 May 2021 (2021-05-28)<br> claims 1-15, description [0001]-[0055], figures 1-7 | 1-15208715994 |
| Y | CN 206576918 U (BEIJING JIUSHI SHENKANG MEDICAL TECHNOLOGY CO., LTD.) 24 October 2017 (2017-10-24)<br> description, paragraphs [0043]-[0045] and [0051]-[0064], and figures 1-11 | 1-15208715994 |
| Y | CN 106618676 A (SHANGHAI ACHIEVA MEDICAL SUZHOU CO., LTD.) 10 May 2017 (2017-05-10)<br> description, paragraphs [0017]-[0025], and figure 1 | 1-15 |
| Y | EP 1172073 A1 (CORDIS CORP.) 16 January 2002 (2002-01-16)<br> description paragraph [0057], figure 19 | 11-15 |
| A | CN 109688946 A (NEUROVASC TECHNOLOGIES INC.) 26 April 2019 (2019-04-26)<br> entire document | 1-15 |
| A | CN 109906058 A (NEURAVI LIMITED) 18 June 2019 (2019-06-18)<br> entire document | 1-15 |
| A | US 2016143653 A1 (NEURAVI LTD) 26 May 2016 (2016-05-26)<br> entire document | 1-15 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **13 April 2022** | **20 April 2022** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

<table>
<tr><td colspan="3"></td><td>International application No.<br>**PCT/CN2022/072103**</td></tr>
<tr><td colspan="4">C.     **DOCUMENTS CONSIDERED TO BE RELEVANT**</td></tr>
<tr><td>Category*</td><td colspan="2">Citation of document, with indication, where appropriate, of the relevant passages</td><td>Relevant to claim No.</td></tr>
<tr><td>A</td><td colspan="2">WO 2019079296 A1 (WALLABY MEDICAL INC) 25 April 2019 (2019-04-25)<br>entire document</td><td>1-15</td></tr>
</table>

Form PCT/ISA/210 (second sheet) (January 2015)

EP 4 279 025 A1

**INTERNATIONAL SEARCH REPORT**
Information on patent family members

International application No.

**PCT/CN2022/072103**

| Patent document cited in search report | | | Publication date (day/month/year) | Patent family member(s) | | | Publication date (day/month/year) |
|---|---|---|---|---|---|---|---|
| CN | 112842467 | A | 28 May 2021 | None | | | |
| CN | 206576918 | U | 24 October 2017 | None | | | |
| CN | 106618676 | A | 10 May 2017 | WO | 2018120254 | A1 | 05 July 2018 |
| | | | | US | 2020029984 | A1 | 30 January 2020 |
| | | | | CN | 106618676 | B | 28 July 2020 |
| EP | 1172073 | A1 | 16 January 2002 | None | | | |
| CN | 109688946 | A | 26 April 2019 | JP | 2019526378 | A | 19 September 2019 |
| | | | | US | 2018344337 | A1 | 06 December 2018 |
| | | | | AU | 2019236713 | A1 | 17 October 2019 |
| | | | | AU | 2021236459 | A1 | 14 October 2021 |
| | | | | US | 2020352585 | A1 | 12 November 2020 |
| | | | | US | 2018064454 | A1 | 08 March 2018 |
| | | | | KR | 20190042582 | A | 24 April 2019 |
| | | | | AU | 2017324159 | A1 | 07 March 2019 |
| | | | | CA | 3115329 | A1 | 15 March 2018 |
| | | | | EP | 3509508 | A1 | 17 July 2019 |
| | | | | WO | 2018048679 | A1 | 15 March 2018 |
| | | | | CA | 3035260 | A1 | 15 March 2018 |
| | | | | CN | 113855167 | A | 31 December 2021 |
| | | | | BR | 112019004278 | A2 | 04 June 2019 |
| | | | | CN | 109688946 | B | 01 October 2020 |
| CN | 109906058 | A | 18 June 2019 | BR | 112019004484 | A2 | 28 May 2019 |
| | | | | RU | 2019110141 | A | 08 October 2020 |
| | | | | KR | 20190051008 | A | 14 May 2019 |
| | | | | US | 2020305900 | A1 | 01 October 2020 |
| | | | | IL | 265151 | D0 | 30 May 2019 |
| | | | | ES | 2867599 | T3 | 20 October 2021 |
| | | | | EP | 3509509 | A2 | 17 July 2019 |
| | | | | EP | 3854322 | A1 | 28 July 2021 |
| | | | | CA | 3035706 | A1 | 15 March 2018 |
| | | | | AU | 2017324233 | A1 | 04 April 2019 |
| | | | | MX | 2019002565 | A | 18 September 2019 |
| | | | | US | 2022000504 | A1 | 06 January 2022 |
| | | | | CO | 2019002678 | A2 | 11 June 2019 |
| | | | | JP | 2019526365 | A | 19 September 2019 |
| | | | | WO | 2018046408 | A2 | 15 March 2018 |
| US | 2016143653 | A1 | 26 May 2016 | US | 2019298397 | A1 | 03 October 2019 |
| | | | | JP | 2017535352 | A | 30 November 2017 |
| | | | | WO | 2016083472 | A1 | 02 June 2016 |
| | | | | EP | 3682821 | A1 | 22 July 2020 |
| | | | | HK | 1247066 | A1 | 21 September 2018 |
| | | | | CN | 111743601 | A | 09 October 2020 |
| | | | | CN | 106999196 | A | 01 August 2017 |
| | | | | JP | 2021098113 | A | 01 July 2021 |
| | | | | EP | 3223723 | A1 | 04 October 2017 |
| | | | | ES | 2781184 | T3 | 31 August 2020 |
| | | | | CA | 3006614 | A1 | 01 June 2017 |
| | | | | WO | 2017089424 | A1 | 01 June 2017 |
| | | | | AU | 2016358836 | A1 | 21 June 2018 |

**INTERNATIONAL SEARCH REPORT**
Information on patent family members

| Patent document cited in search report | | | Publication date (day/month/year) | Patent family member(s) | | | Publication date (day/month/year) |
|---|---|---|---|---|---|---|---|
| | | | | KR | 20180087320 | A | 01 August 2018 |
| | | | | CN | 108601599 | A | 28 September 2018 |
| | | | | EP | 3380021 | A1 | 03 October 2018 |
| | | | | BR | 112018010607 | A2 | 13 November 2018 |
| | | | | MX | 2018006446 | A1 | 06 June 2019 |
| | | | | US | 10363054 | B2 | 30 July 2019 |
| | | | | CN | 106999196 | B | 28 July 2020 |
| | | | | CN | 108601599 | B | 13 August 2021 |
| | | | | JP | 6961590 | B2 | 05 November 2021 |
| WO | 2019079296 | A1 | 25 April 2019 | US | 2020297364 | A1 | 24 September 2020 |
| | | | | CN | 111246811 | A | 05 June 2020 |
| | | | | JP | 2020536607 | A | 17 December 2020 |
| | | | | EP | 3697326 | A1 | 26 August 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 202110053305